# EUROPÄISCHE PATENTANMELDUNG

(11) **EP 0 275 417 A2**
(43) Veröffentlichungstag der Anmeldung: **27.07.1988**
(21) Anmeldenummer: 87117634.3
(22) Anmeldetag: 28.11.1987
(51) Int. Cl.: C07D 263/42, C07D 233/70, C07D 277/34, C07D 413/12, C07D 417/12, C07D 417/04, C07D 413/04, C07D 417/14, C07D 413/14, A01N 43/76, A01N 43/78

(54) **Trifluormethylsubstituierte Azole, Verfahren zu ihrer Herstellung und ihre Verwendung als Mittel mit herbizider Wirkung**

(30) Priorität: 02.12.1986 DE 3641229
(71) Anmelder: SCHERING AKTIENGESELLSCHAFT, D-13342 Berlin (DE)
(72) Erfinder: Hübl, Dieter, Dr., D-1000 Berlin 41 (DE); Ganzer, Michael, Dr., D-1000 Berlin 51 (DE); Arndt, Friedrich, Dr., D-1000 Berlin 28 (DE); Rees, Richard, Dr., D-1000 Berlin 28 (DE)

(57) **Zusammenfassung**

Die Erfindung betrifft neue trifluormethylsubstituierte Azole der allgemeinen Formel I 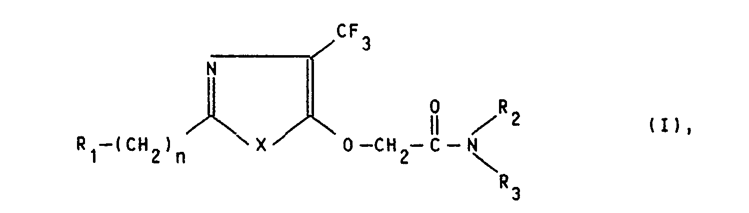 in der R₁, R₂, R₃, X und n die in der Beschreibung genannten Bedeutungen haben, ein Verfahren zu ihrer Herstellung und ihre Verwendung zur Bekämpfung von unerwünschtem Pflanzenwuchs in Nutzpflanzenkulturen.

## Beschreibung

Die Erfindung betrifft neue trifluormethylsubstituierte Azole, Verfahren zu ihrer Herstellung und ihre Verwendung als Mittel mit herbizider Wirkung.

Es ist bereits bekannt, daß Azolyloxyacetamide herbizide Eigenschaften besitzen (DE-OS 29 14 003, DE-OS 32 18 482, DE-OS 32 28 131 und EP-Anmeldung 0 161 602). Die Herbizidwirkung der bekannten Verbindungen ist häufig jedoch nicht ausreichend.

Aufgabe der vorliegenden Erfindung ist es, ein Unkrautmittel zu schaffen, welches außer der Wirkung gegen einige wichtige breitblättrige Unkräuter und einer hervorragenden Wirksamkeit gegen Ungräser auch ein breites Selektivitätsspektrum vor allem in dikotylen Kulturen und bei Getreidearten zeigt.

Diese Aufgabe wird erfindungsgemäß gelöst durch die Bereitstellung von neuen trifluormethylsubstituierten Azolen der allgemeinen Formel I 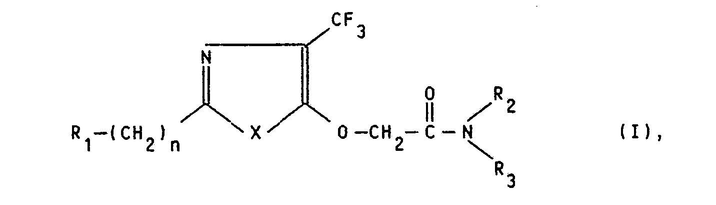 in der
R₁ einen gegebenenfalls ein- oder mehrfach, gleich oder verschieden durch Halogen, C_{₁-₄}-Alkoxy, C₁₋₄-Alkylthio oder Di-C₁₋₄-alkylaminosubstituierten C₁₋₁₂-Alkyl-, C₂₋₁₂-Alkenyl- oder C₂₋₁₂-Alkinylrest, einen C₃₋₈-Cycloalkylrest, einen C₁₋₆-Alkoxy-, C₁₋₆-Alkylthio- oder Di-C₁₋₆-alkylaminorest, einen gegebenenfalls ein- oder mehrfach, gleich oder verschieden durch Halogen, C₁₋₄-Alkyl, C₁₋₄-Alkoxy, C₁₋₄-Alkylthio, Di-C₁₋₄-alkylamino, Halogen-C₁₋₄-alkyl, Halogen-C₁₋₄-alkoxy, Halogen-C₁₋₄-alkylthio, C₁₋₆-Alkoxycarbonyl, Halogen-C₁₋₆-alkoxycarbonyl, Nitro, Cyan, Phenoxy, Halogenphenoxy, Phenylthio oder Halogenphenylthio substituierten Phenyl-, Biphenyl-, Naphthyl-, Furyl-, Thienyl-, Pyridyl-, Benzofuryl-, Benzothienyl-, Chinolinyl-, Isochinolinyl-, Phenoxy- oder Phenylthiorest,
R₂ und R₃ unabhängig voneinander ein Wasserstoffatom, einen gegebenenfalls ein- oder mehrfach, gleich oder verschieden durch Halogen, C₁₋₄-Alkoxy, C₁₋₄-Alkylthio oder Di-C₁₋₄-Alkylamino substituierten C₁₋₈-Alkyl-, C₂₋₈-Alkenyl- oder C₂₋₈-Alkinyl- rest, einen C₃₋₈-Cycloalkyl- oder C₃₋₈-Cycloalkenylrest, einen gegebenenfalls durch ein oder mehrere Sauerstoff-, Stickstoff- oder Schwefelatome unterbrochenen C₁₋₁₂-Alkyl-, C₃₋₈-Cycloalkyl-, C₃₋₈-Cycloalkenyl-, C₃₋₈-Cycloalkyl-C₁₋₄-alkyl- oder C₃₋₈-Cycloalkenyl-C₁₋₄-alkylrest, einen C₁₋₈-Alkoxyrest, einen gegebenenfalls ein- oder mehrfach, gleich oder verschieden durch Halogen, C₁₋₄-Alkyl, C₁₋₄-Alkoxy, C₁₋₄-Alkylthio, Di-C₁₋₄-alkylamino, Halogen-C₁₋₄-alkyl, Halogen-C₁₋₄-alkoxy, Halogen-C₁₋₄-alkylthio, C₁₋₆-Alkoxycarbonyl, Halogen-C₁₋₆-alkoxycarbonyl, Nitro, Cyan, Phenoxy, Halogenphenoxy, Phenylthio oder Halogenphenylthio substituierten Phenyl-, Biphenyl-oder Naphthylrest oder
R₂ und R₃ zusammen mit dem benachbarten Stickstoffatom einen gegebenenfalls weitere Heteroatome wie Sauerstoff, Stickstoff oder Schwefel enthaltenden und gegebenenfalls ein- oder mehr fach, gleich oder verschieden durch Halogen oder C₁₋₄-Alkyl substituierten gesättigten oder ungesättigten Monocyclus oder anellierten Bicyclus,
n 0, 1 oder 2,
x ein Sauerstoff- oder Schwefelatom oder die Gruppe NR₄ und
R₄ einen C₁₋₁₂-Alkylrest oder einen gegebenenfalls ein- oder mehrfach, gleich oder verschieden durch Halogen, C₁₋₄-Alkyl, C₁₋₄-Alkoxy, C₁₋₄-Alkylthio, Di-C₁₋₄-alkylamino, Halogen-C₁₋₄-alkyl, Halogen-C₁₋₄-alkoxy, Halogen-C₁₋₄-alkylthio, C₁₋₆-Alkoxycarbonyl, Halogen-C₁₋₆-alkoxycarbonyl, Nitro, Cyan, Phenoxy, Halogenphenoxy, Phenylthio, Halogenphenylthio substituierten Phenyl-, Biphenyl-, Naphthyl-, Furyl-, Thienyl-, Pyridyl-, Benzofuryl-, Benzothienyl-, Chinolinyl- oder Isochinolinylrest
bedeuten.

Unter "Halogen" ist Fluor, Chlor, Brom oder Jod zu verstehen.

Die Bezeichnung "Halogenalkyl" besagt, daß ein oder mehrere Wasserstoffatome des Alkylrestes durch Halogen ersetzt sind.

Als Mono- beziehungsweise Bicyclen seien als Beispiele genannt: 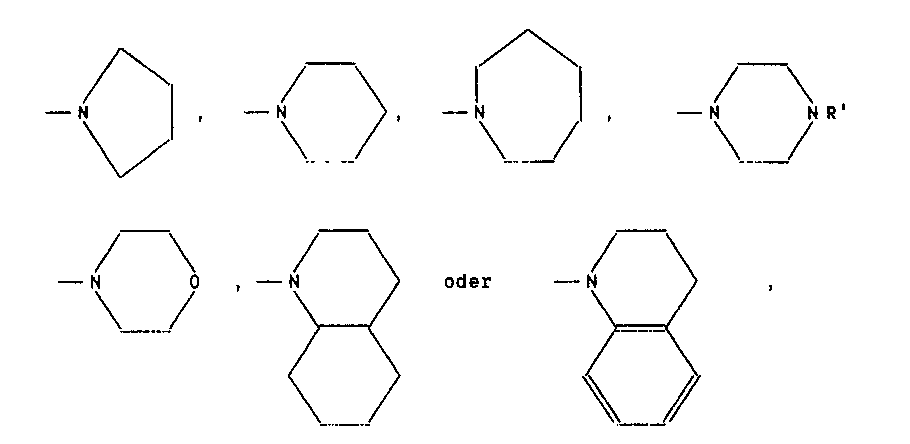 worin Rʹ ein Wasserstoffatom, einen C₁₋₄-Alkylrest, einen gegebenenfalls ein- oder mehrfach, gleich oder verschieden durch Halogen, C₁₋₄-Alkyl, C₁₋₄-Alkoxy, C₁₋₄-Alkylthio, Halogen-C₁₋₄-alkyl, Halogen-C₁₋₄-alkoxy, Halogen-C₁₋₄-alkylthio oder Nitro substituierten Phenyl- oder Benzylrest bedeutet.

Besonders bevorzugt sind die Verbindungen der allgemeinen Formel I, in denen
R₁ einen Phenylrest, einen Halogenphenylrest, einen Methylphenylrest, einen Methoxyphenylrest oder einen Furylrest,
R₂ und R₃ unabhängig voneinander einen C₁₋₄-Alkylrest, einen C₂₋₄-Alkenylrest oder einen Phenylrest oder
R₂ und R₃ zusammen mit dem benachbarten Stickstoffatom einen gegebenenfalls durch C₁₋₄-Alkyl substituierten Piperidino- oder Hexahydroazepinylrest,
n 0,
X ein Sauerstoff- oder Schwefelatom oder die Gruppe NR₄ und
R₄ Dimethylphenyl
bedeuten.

Die erfindungsgemäßen Verbindungen der allgemeinen Formel I lassen sich zum Beispiel herstellen, indem man 5-fluor-4-(trifluormethyl)-substituierte Azole der allgemeinen Formel II 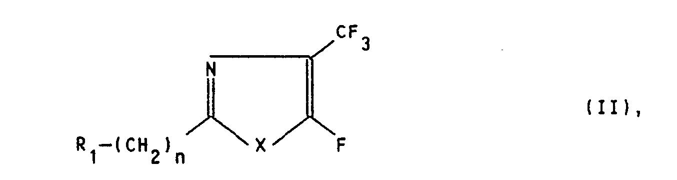 in der R₁, X und n die oben geannten Bedeutungen haben, mit Hydroxyessigsäureamiden der allgemeinen Formel III 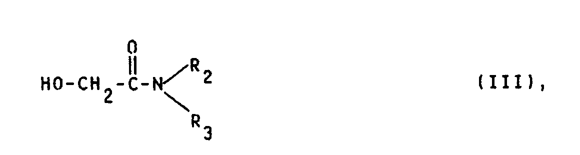 in der R₂ und R₃ die oben genannten Bedeutungen haben, umsetzt.

Die Umsetzung wird zweckmäßigerweise so durchgeführt, daß man die Verbindungen der allgemeinen Formel II in inerten Lösungsmitteln, wie zum Beispiel Dioxan, Tetrahydrofuran oder Ether, im Temperaturbereich zwischen 20°C und 100°C mit Hydroxyessigsäureamiden der allgemeinen Formel III im Moverhältnis 1:1 bis 1:3, bezogen auf die Verbindungen der allgemeinen Formel II, unter Basenzusatz, wie zum Beispiel Natriumhydrid, Lithiumhydroxid oder Triethylamin, zur Reaktion bringt. Die Reaktionsdauer beträgt ca. 0,5 bis 20 Stunden.

Das Reaktionsgemisch wird nach Abtrennen der ungelösten Anteile zur Trockene eingedampft. Der Rückstand wird mit Wasser versetzt und mehrmals mit Diethylether, Chloroform, Dichlormethan oder Essigester extrahiert. Die organischen Lösungsmittelauszüge werden über Magnesiumsulfat getrocknet und eingeengt.

Die anfallenden Rohprodukte lassen sich in üblicher Weise durch Umkristallisation, Vakuumdestillation oder Säulenchromatographie reinigen.

Die Ausgangsmaterialien der allgemeinen Formeln II und III zur Herstellung der erfindungsgemäßen Verbindungen sind teilweise bekannt oder lassen sich nach bekannten Verfahren herstellen.

Die erfindungsgemäßen Verbindungen stellen in der Regel farb- und geruchlose Kristalle oder zähflüssige Substanzen dar, die in Wasser schwerlöslich und in organischen Lösungsmitteln mehr oder weniger gut löslich sind.

Die gekennzeichneten Verbindungen zeichnen sich durch eine breite blatt- und bodenherbizide Wirkung gegen Monokotyle aus. Sie können zur Bekämpfung mono- und dikotyler Unkräuter eingesetzt werden. Auch die Einarbeitung in den Boden ermöglicht eine gute Unkrautbekämpfung.

Mit den erfindungsgemäßen Verbindungen werden Unkräuter der Gattungen Avena, Alopecurus, Echinochloa, Setaria, Panicum, Digitaria, Poa, Eleusine, Brachiaria, Lolium, Bromus, Cyperus, Agropyron, Sagittaria, Cynodon, Monochoria, Fimbristylis, Eleocharis, Ischaemum, Apera, Holcus, Matricaria und Stellaria gut bekämpft.

Die Verwendung der erfindungsgemäßen Wirkstoffe ist jedoch keineswegs auf die genannten Gattungen beschränkt.

Zur Bekämpfung von Unkräutern werden in der Regel Aufwandmengen von 0,1 kg Wirkstoff/ha bis 5 kg Wirkstoff/ha verwendet. Dabei erweisen sich die gekennzeichneten Mittel selektiv in Nutzpflanzenkulturen wie Baumwolle, Erdnuß, Raps, Markstammkohl, Luzerne, Blumenkohl, Sojabohne, Erbse, Zuckerrübe, Spinat, Salat, Sonnenblume, Mais, Weizen und Reis.

Die erfindungsgemäß zu verwendenden Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden.

Sofern eine Verbreiterung des Wirkungsspektrums beabsichtigt ist, können auch andere Herbizide zugesetzt werden. Beispielsweise eignen sich als herbizid wirksame Mischungspartner diejenigen Wirkstoffe, die in Weed Abstracts, Vol. 35, No.5, 1986, unter dem Titel "List of common names and abbreviations employed for currently used herbicides and plant growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Als Mischungspartner können außerdem Phospholipide verwendet werden, zum Beispiel solche aus der Gruppe Phosphatidylcholin, den hydrierten Phosphatidylcholinen, Phosphatidylethanolamin, den N-Acyl-phosphatidylethanolaminen, Phosphatidylinosit, Phosphatidylserin, Lysolecithin und Phosphatidylglycerol.

Zweckmäßig werden die gekennzeichneten Wirkstoffe oder deren Mischungen in Form von Zubereitungen wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier-und/oder Dispergierhilfsmitteln angewandt.

Geeignete flüssige Trägerstoffe sind zum Beispiel aliphatische und aromatische Kohlenwasserstoffe wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen von etwa 100 bis 1000 Liter/- ha. Eine Anwendung der Mittel im sogenannten Low-Volume und Ultra-Low-Volume-Verfahren ist ebenso möglich wie ihre Applikation in Form von sogennanten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

### A) Spritzpulver

1.)
20 Gewichtsprozent Wirkstoff
68 Gewichtsprozent Kaolin
10 Gewichtsprozent Calciumsalz der Ligninsulfonsäure
2 Gewichtsprozent Dialkylnaphthalinsulfonat

2.)
40 Gewichtsprozent Wirkstoff
25 Gewichtsprozent Kaolin
25 Gewichtsprozent kolloidale Kieselsäure
8 Gewichtsprozent Calciumsalz der Ligninsulfonsäure
2 Gewichtsprozent Natriumsalz des N-Methyl-N-oleyl-taurins

### B) Paste

45 Gewichtsprozent Wirkstoff
5 Gewichtsprozent Natriumaluminiumsilikat
15 Gewichtsprozent Cetylpolyglycolether mit 8 Mol Ethylenoxid
2 Gewichtsprozent Spindelöl
10 Gewichtsprozent Polyethylenglycol
23 Gewichtsprozent Wasser

### C) Emulsionskonzentrat

20 Gewichtsprozent Wirkstoff
75 Gewichtsprozent Isophoron
2 Gewichtsprozent ethoxyliertes Rizinusöl
3 Gewichtsprozent Calciumsalz der Dodecylphenylsulfonsäure

Die folgenden Beispiele beschreiben die Herstellung der erfindungsgemäßen Verbindungen:

### Beispiel 1

### 2-[2-(4-Fluorphenyl)-4-trifluormethyl-5-oxazolyloxy]-essigsäure-dimethylamid

Zu einer Mischung bestehend aus 0,82 g (27,5 mmol) Natriumhydrid (in Paraffin 80 %ig) und 2,83 g (27,5 mmol) Hydroxyessigsäuredimethylamid in 20 ml wasserfreiem Tetrahydrofuran wird eine Lösung von 6,23 g (25 mmol) 5-Fluor-2-(4-fluorphenyl)-4-trifluormethyloxazol in 20 ml wasserfreiem Tetrahydrofuran langsam zugetropft. Das Gemisch wird 5 Stunden bei Raumtemperatur gerührt. Danach wird das Reaktionsgemisch im Vakuum bis zur Trockne eingeengt und der Rückstand in 100 ml Diethylether aufgenommen. Die Etherphase wird dreimal mit je 25 ml Wasser gewaschen, über Magnesiumsulfat getrocknet, filtriert und eingeengt. Die Umkristallisation des Rückstandes erfolgt aus Hexan.
Ausbeute: 4,3 g = 51,7 % der Theorie
Fp.: 121 °C
Elementaranalyse:
ber. C 50,61% H 3,64 % N 8,48 % F 22,87 %
gef. C 50,66% H 3,76 % N 8,60 % F 23,19 %

### Herstellung des Ausgangsmaterials

### 5-Fluor-2-(4-fluorphenyl)-4-trifluormethyloxazol

Eine Lösung von 2,88 g (10 mmol) 4-Fluorbenzoesäure-[bis-(trifluormethyl)-methylen]-amid in 30 ml wasserfreiem Toluol wird durch Zugabe von 2,84 g (15 mmol) wasserfreiem Zinn-(II)-chlorid 8 Stunden bei 140 °C (Badtemperatur) unter Stickstoffatmosphäre kräftig gerührt. Nach Abtrennen der ungelösten Anteile wird im Vakuum zur Trockne eingedampft und der Rückstand mehrmals mit heißem Hexan extrahiert. Die Lösungsmittelauszüge werden eingeengt und anschließend säulenchromatographiert (Eluent: Hexan).
Ausbeute: 1,79 g = 71,9 % der Theorie
Kp.: 29 °C / 0,1 Torr
n 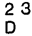 : 1,4692
Elementaranalyse:
ber. C 48,21 % H 1,62 % N 5,62 %
gef. C 48,34 % H 1,88 % N 5,68 %

### Beispiel 2

### (2-Ethylpiperidino)-[2-(4-methoxyphenyl)-4-trifluormethyl-5-thiazolyloxymethyl]-keton

Zu einer Mischung bestehend aus 0,90 g (30 mmol) Natriumhydrid (in Paraffin 80 %ig) und 5,14 g (30 mmol) (2-Ethylpiperidino)-hydroxymethylketon in 20 ml wasserfreiem Tetrahydrofuran wird eine Lösung von 7,62 g (27,5 mmol) 5-Fluor-2-(4-methoxyphenyl)-4-trifluormethylthiazol in 20 ml wasserfreiem Tetrahydrofuran langsam zugetropft. Das Gemisch wird 3 Stunden bei Raumtemperatur gerührt. Das Reaktionsgemisch wird anschließend im Vakuum bis zur Trockne eingeengt und der Rückstand in 200 ml Diethylether aufgenommen. Die Etherphase wird dreimal mit je 50 ml Wasser gewaschen, über Magnesiumsulfat getrocknet, filtriert und eingeengt. Die Umkristallisation des Rückstands erfolgt aus Hexan.
Ausbeute: 9,6 g = 81.5 % der Theorie
Fp.: 73-74 °C
Elementaranalyse:
ber. C 56,06 % H 5,41 % N 6,54 %
gef. C 55,97 % H 5,28 % N 6,22 %

### Herstellung des Ausgangsmaterials

### 5-Fluor-2-(4-methoxyphenyl)-4-trifluormethylthiazol

Eine Lösung von 67,38 g (140 mmol) 4-(4-Methoxyphenyl)-2,2,6,6-tetrakis-(trifluormethyl)-6H-1,3,5-oxathiazin in 350 ml wasserfreiem Xylol wird nach Zugabe von 32,2 g (170 mmol) wasserfreiem Zinn(II)-chlorid 20 Stunden bei 140 °C (Badtemperatur) unter Stickstoffatmosphäre kräftig gerührt. Nach Abtrennen der ungelösten Anteile wird im Vakuum zur Trockne eingedampft und der Rückstand mehrmals mit heißem Hexan extrahiert. Die Lösungsmittelauszüge werden eingeengt und anschließend säulenchromatographiert (Eluent: Hexan).
Ausbeute: 17,5 g = 45,1 % der Theorie
Fp.: 39-40 °C
Elementaranalyse:
ber. C 47,66 % H 2,55 % N 5,05 %
gef. C 47,76 % H 2,66 % N 5,11 %

### Beispiel 3

### 2-[2-Phenyl-4-trifluormethyl-1-(2,6-xylyl)-5-imidazolyloxy]-essigsäuredimethlamid

Zu einer Mischung bestehend aus 1,00 g (33 mmol) Natriumhydrid (in Paraffin 80 %ig) und 3,20 g (31 mmol) Hydroxyessigsäuredimethylamid in 20 ml wasserfreiem Tetrahydrofuran wird eine Lösung von 9,30 g (27,8 mmol) 1-(2,6-Dimethylphenyl)-5-fluor-2-phenyl-4-(trifluormethyl)-imidazol in 30 ml wasserfreiem Tetrahydrofuran langsam zugetropft. Das Gemisch wird 2 Stunden bei Raumtemperatur gerührt. Das Reaktionsgemisch wird bis zur Trockne einrotiert und der Rückstand in 200 ml Essigsäureethylestser aufgenommen. Die organische Phase wird zweimal mit je 50 ml Wasser gewaschen, über Magnesiumsulfat getrocknet, filtriert und eingeengt. Das zurückbleibende Öl wird anschließend säulenchromatographiert (Eluent: Hexan/Essigsäureethylester 1:1).
Ausbeute: 3,0 = 25,9 % der Theorie
Fp.: 99-100 °C
Elementaranalyse:
ber. C 63,30 % H 5,31 % N 10,07 %
gef. C 63,53 % H 5,10 % N 10,05 %

### Herstellung des Ausgangsmaterials

### 5-Fluor-2-phenyl-4-trifluormethyl-1-(2,6-xylyl)-imidazol

Eine Lösung von 20,00 g (53,7 mmol) N¹-[Bis-(trifluormethyl)-methylen]-N²-(2,6-xylyl)-benzamidin in 150 ml wasserfreiem Xylol wird nach Zugabe von 10,24 g (54 mmol) wasserfreiem Zinn(II)-chlorid 3 Stunden bei 140 °C (Badtemperatur) unter Stickstoffatmosphäre kräftig gerührt. Nach Abtrennen der ungelösten Anteile wird im Vakuum zur Trockne eingedampft und der Rückstand mehrmals mit heißem Hexan extrahiert. Die Hexanauszüge werden eingeengt und anschließend säulenchromatographiert (Eluent: Hexan).
Ausbeute: 16,9 g = 93,6 % der Theorie
Fp.: 79-81 °C
Elementaranalyse:
ber. C 64,67 % H 4,22 % N 8,38 %
gef. C 64,68 % H 3,98 % N 8,29 %

In analoger Weise wurden auch die folgenden Verbindungen hergestellt:

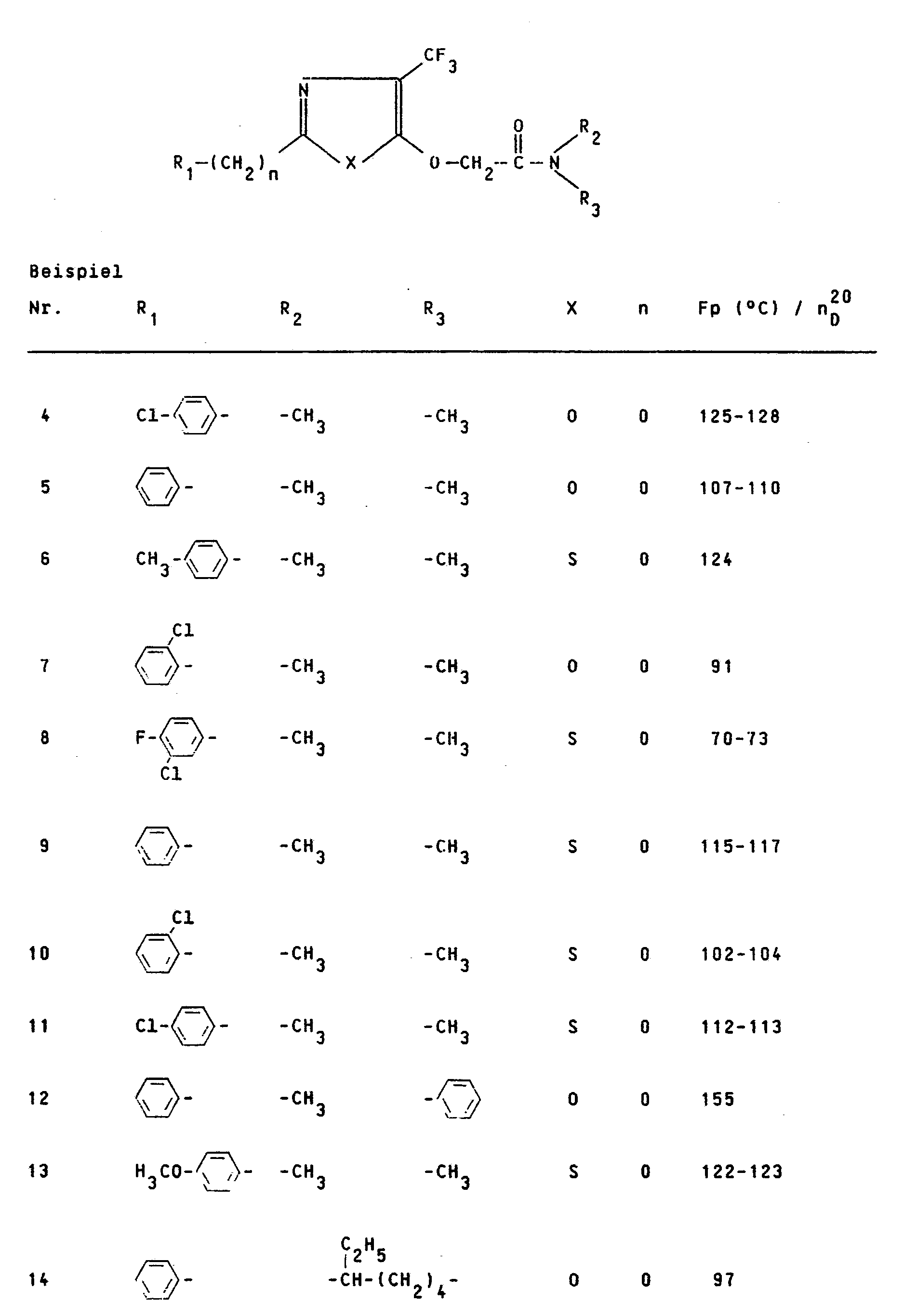

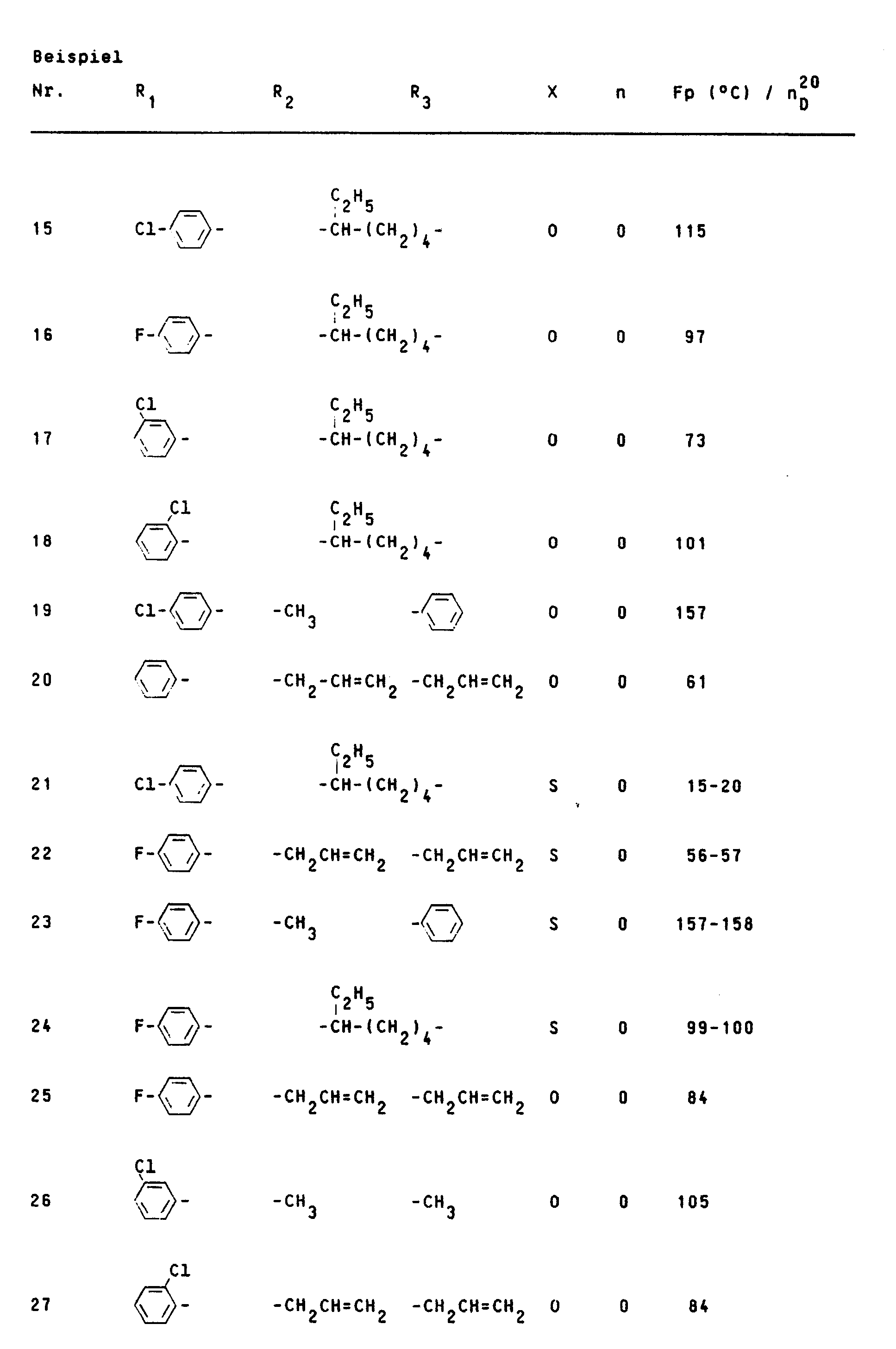

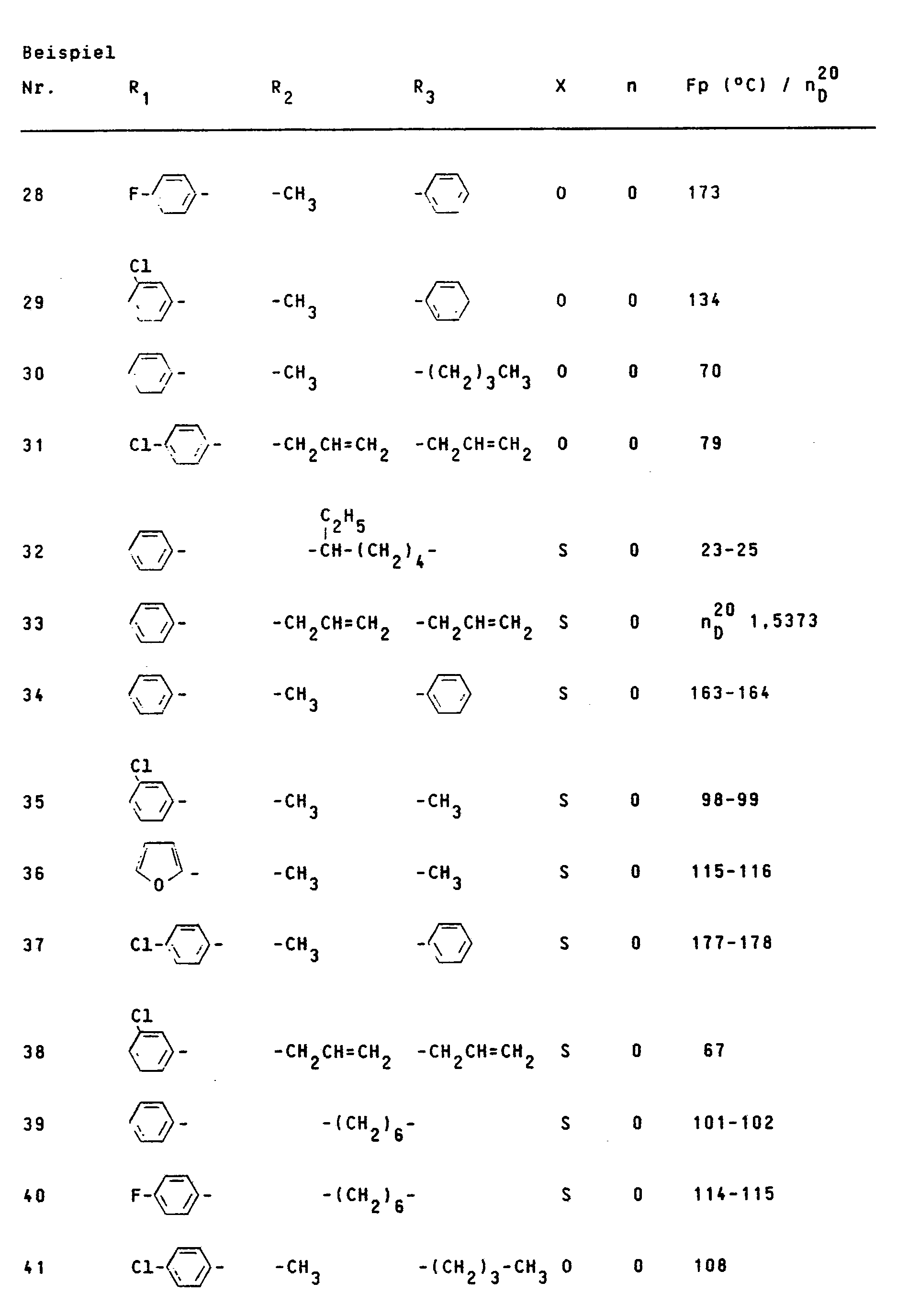

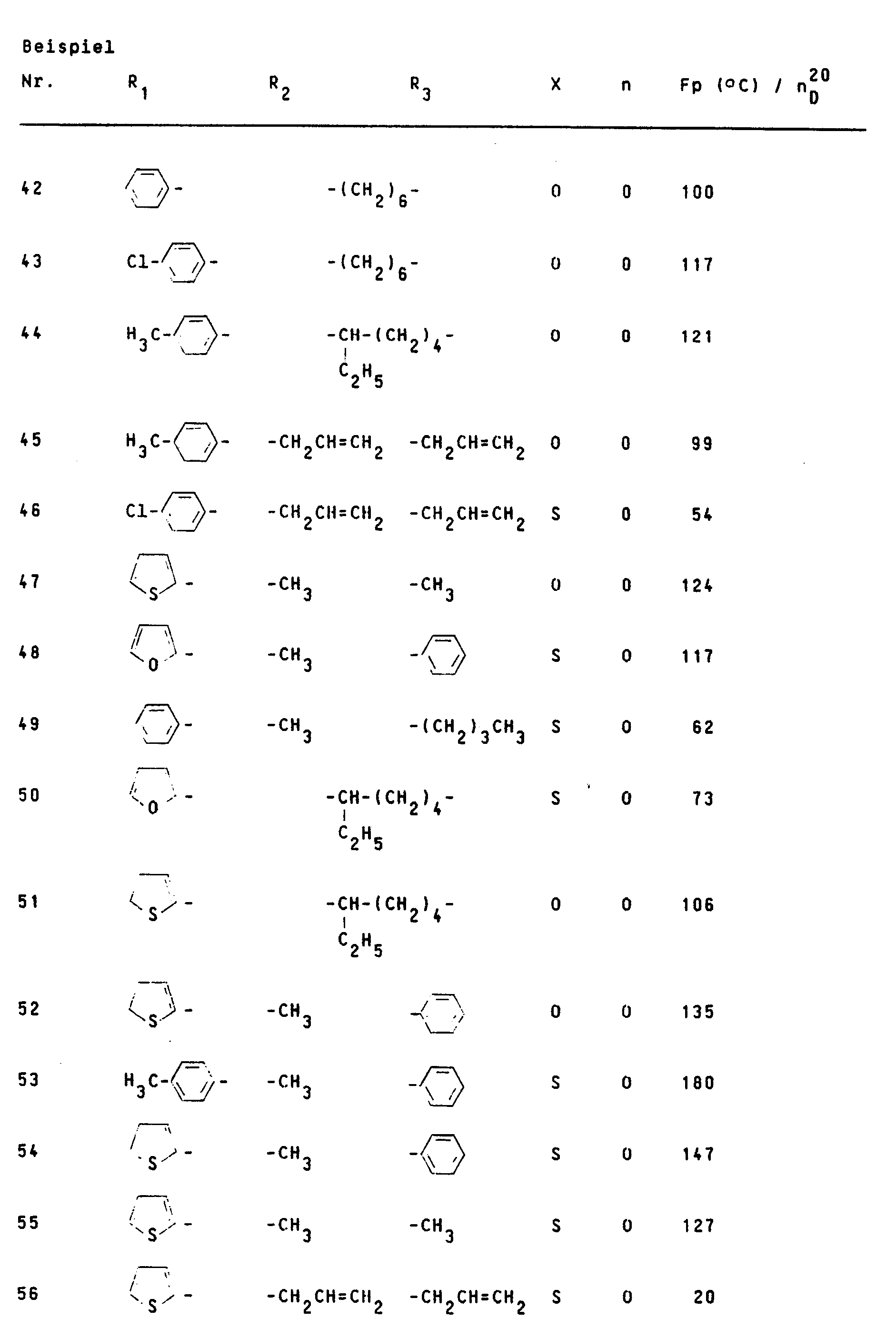

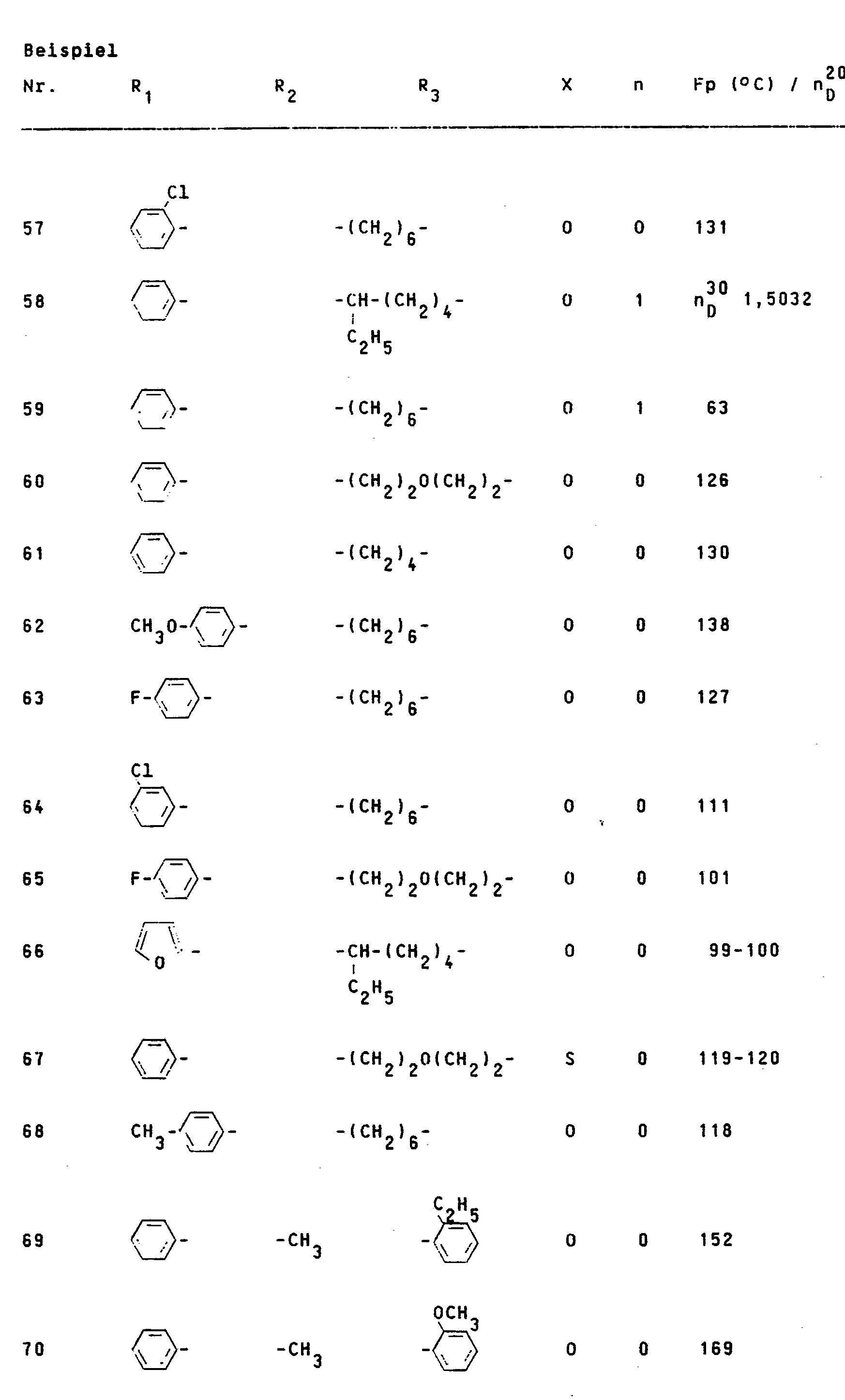

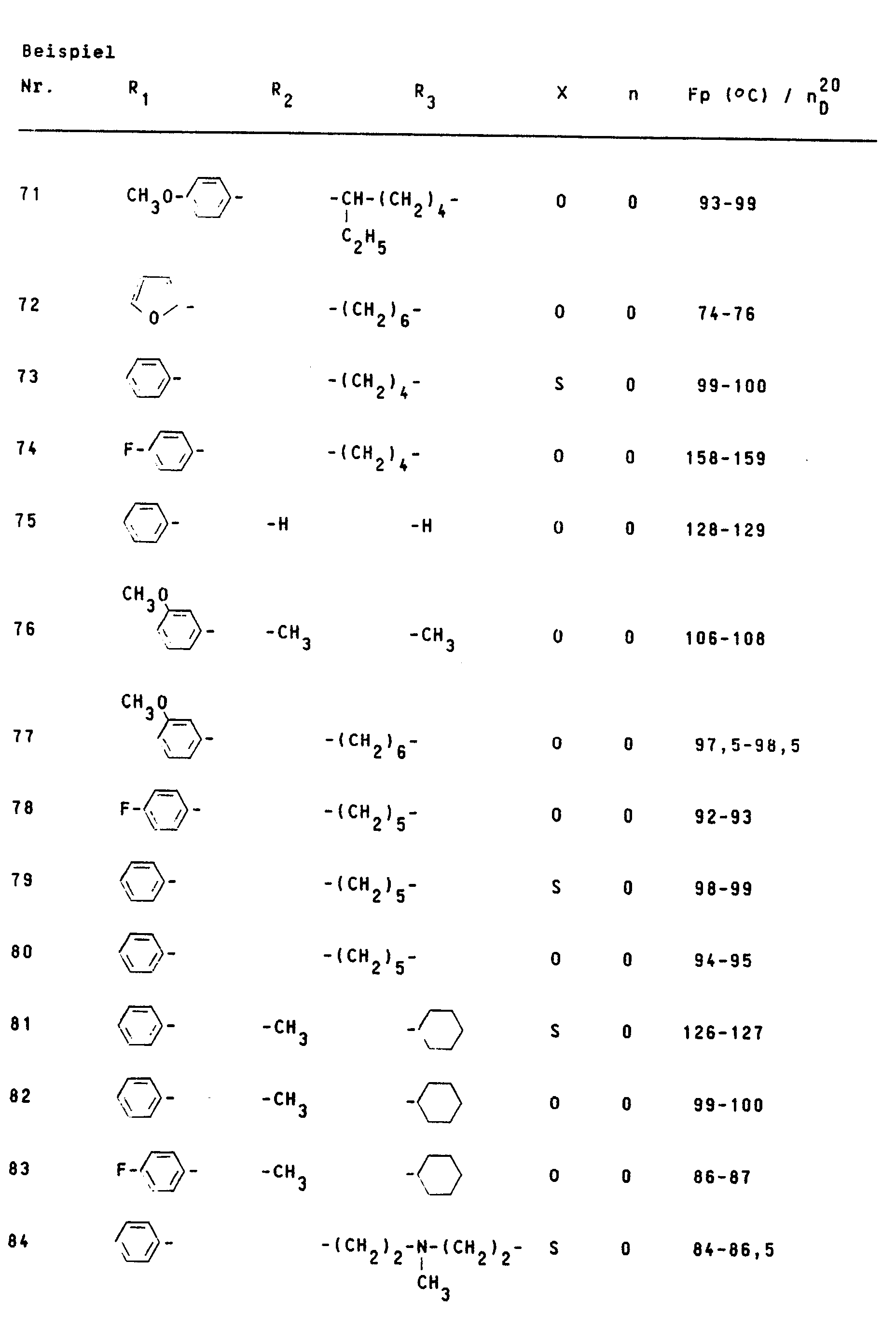

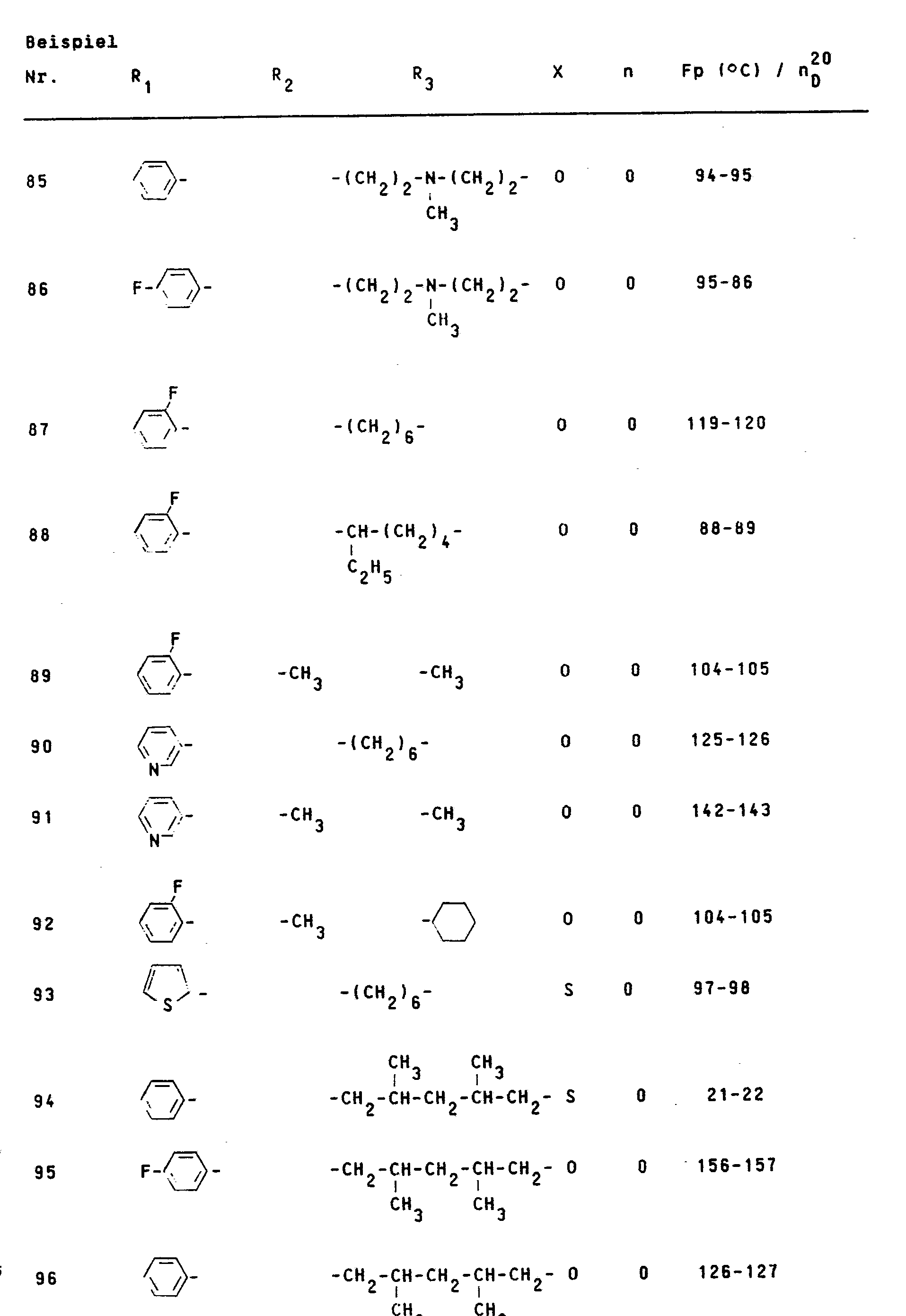

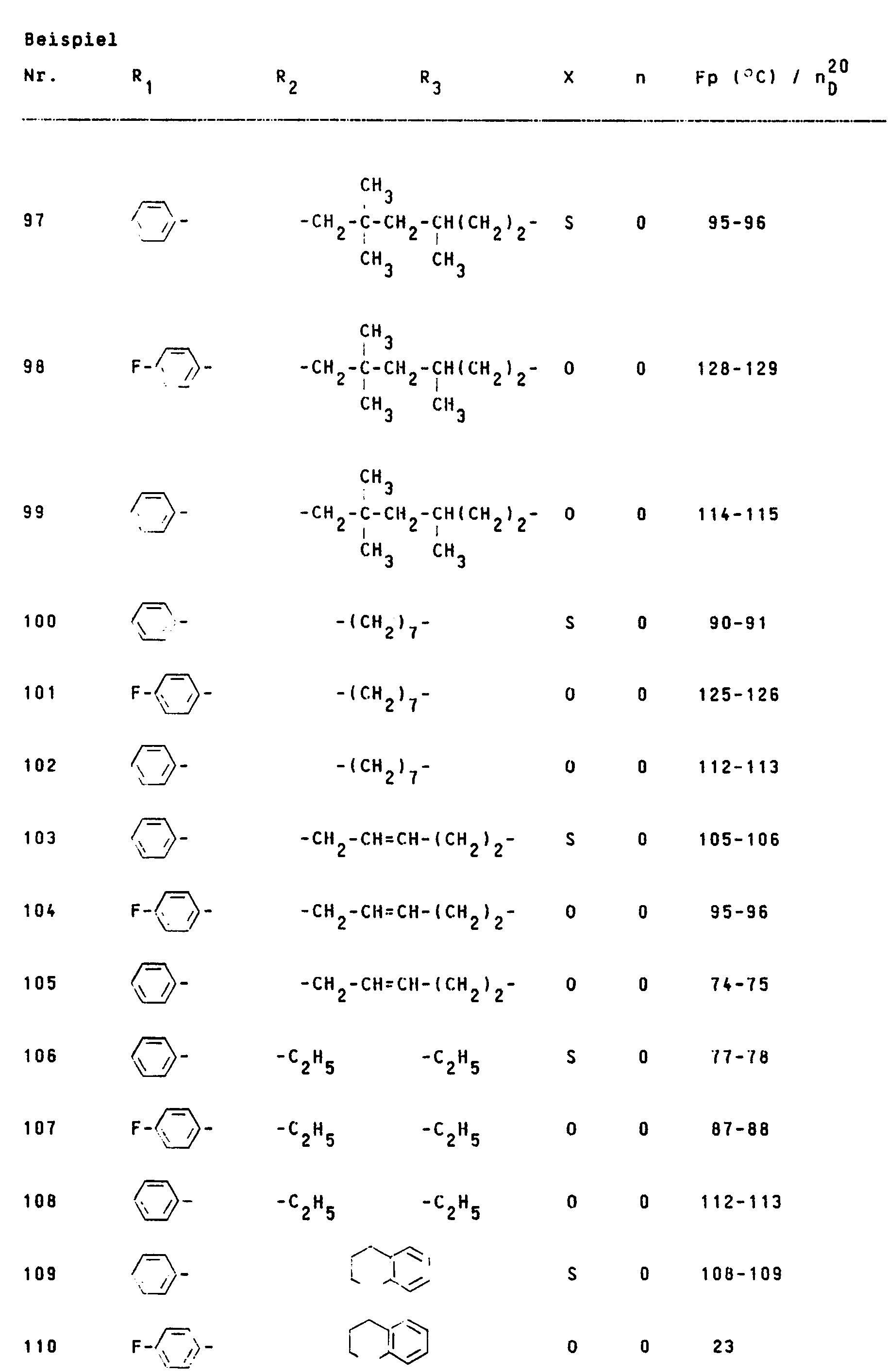

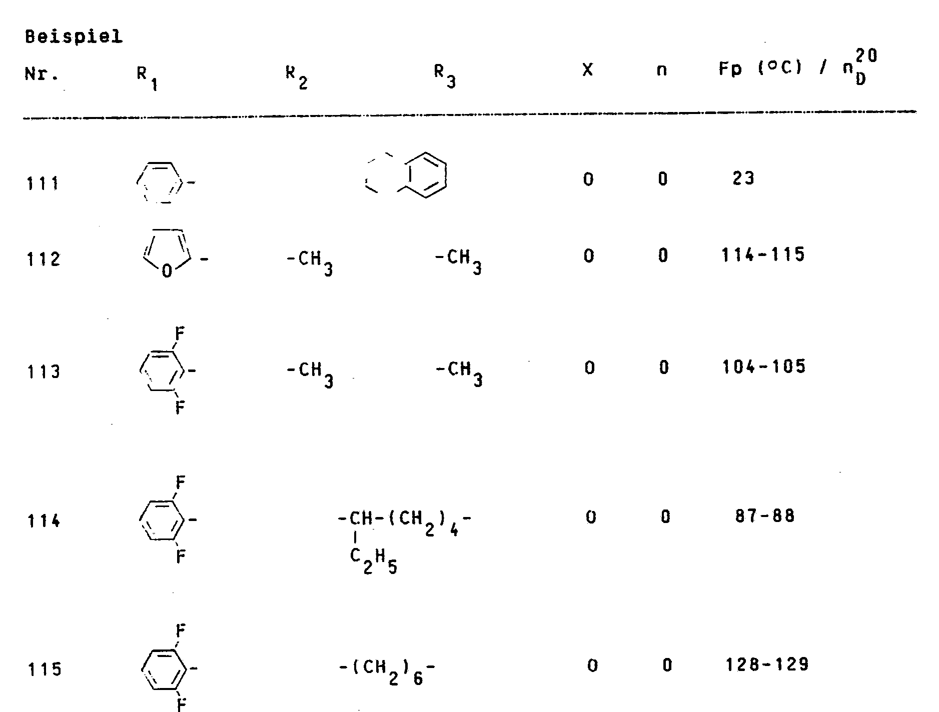

Die folgenden Beispiele erläutern die Anwendungsmöglichkeiten der erfindungsgemäßen Verbindungen:

### Beispiel A

Im Gewächshaus wurden die aufgeführten Pflanzenspezies vor dem Auflaufen mit den aufgeführten Mitteln in einer Aufwandmenge von 3 kg Wirkstoff/ha behandelt. Drei Wochen nach der Behandlung zeigten die erfindungsgemäßen Mittel eine hohe Selektivität bei ausgezeichneter Wirkung gegen Ungräser und Unkräuter. Das Vergleichsmittel zeigte diese selektiven Eigenschaften beziehungsweise diese Wirkung nicht.

In der folgenden Tabelle bedeuten:
0 = keine Wirkung
4 = Vernichtung der Pflanzen

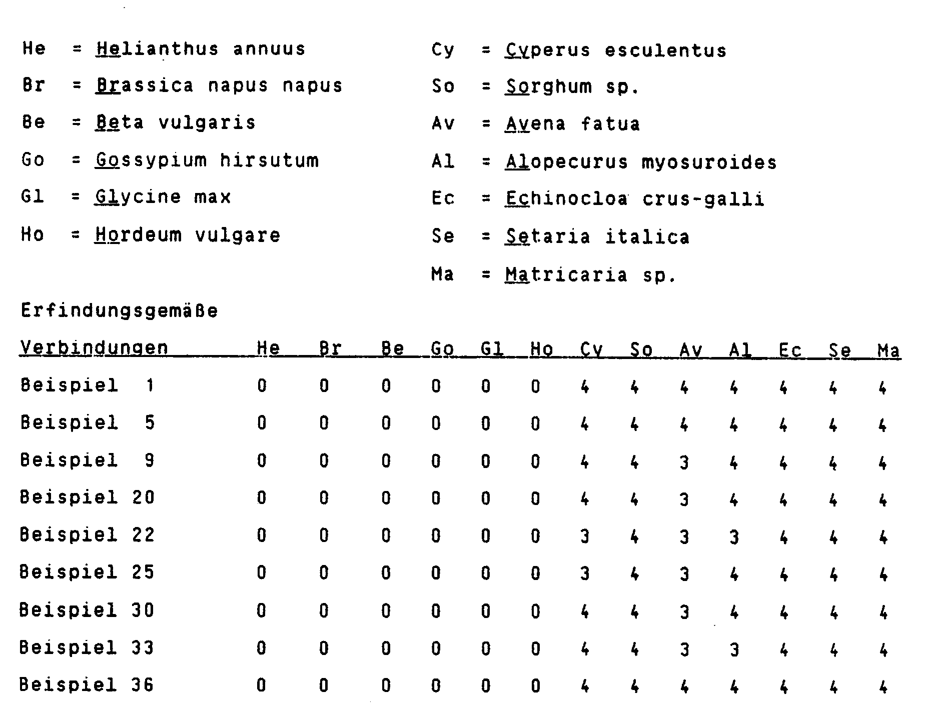

### Vergleichsmittel

Ähnliche herbizide Wirkungen zeigten auch die erfindungsgemäßen Verbindungen gemäß den Beispielen 2 bis 4, 6 bis 8, 10 bis 19, 21, 23, 24, 26 bis 29, 31, 32, 34, 35 und 37 bis 115.

### Beispiel B

Im Gewächshaus wurden die in der Tabelle aufgeführten erfindungsgemäßen Verbindungen in einer Aufwandmenge von 3 kg Wirkstoff/ha, emulgiert in 500 Litern Wasser/ha, auf Avena fatua, Alopecurus myosuroides, Beta vulgaris und auf Brassica napus napus im Nachauflauf gespritzt. Drei Wochen nach der Behandlung wurde das Behandlungsergebnis bonitiert. Wie aus der Tabelle ersichtlich wird, wurden durch die erfindungsgemäßen Verbindungen die Ungräser unter Schonung der Kultur bekämpft.

In der folgenden Tabelle bedeuten:
0 = keine Wirkung
4 = Vernichtung der Pflanzen

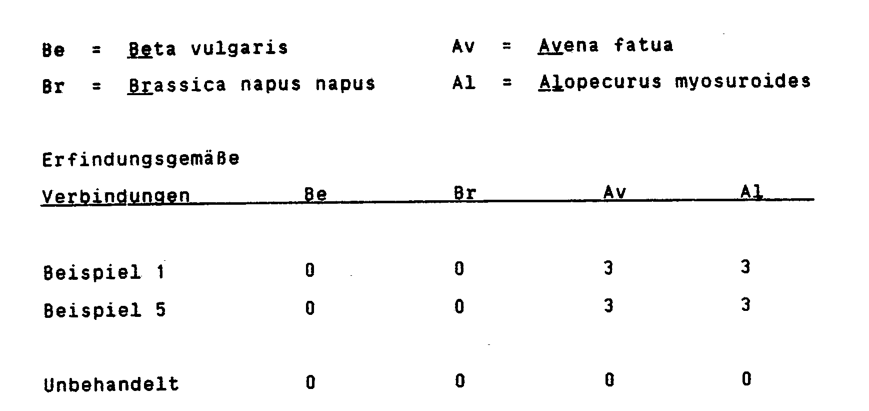

### Beispiel C

Im Gewächshaus wurden die in der Tabelle aufgeführten Verbindungen mit den ebenfalls erwähnten Aufwandmengen appliziert. Hierzu wurden die Wirkstoffe in Gefäße mit 1500 ml Wasser gegeben. Als Testpflanzen wurden Echinochloa crus-galli, Cyperus esculentus, Fimbristylis miliacea und Cyperus difformis eingesetzt. Drei Wochen nach der Applikation wurde die Schädigung der Pflanzen bonitiert.

In der folgenden Tabelle bedeuten:
0 = keine Wirkung
1 = schwache Schädigung
2 = mittlere Schädigung
3 = starke Schädigung
4 = total vernichtet
- = nicht getestet

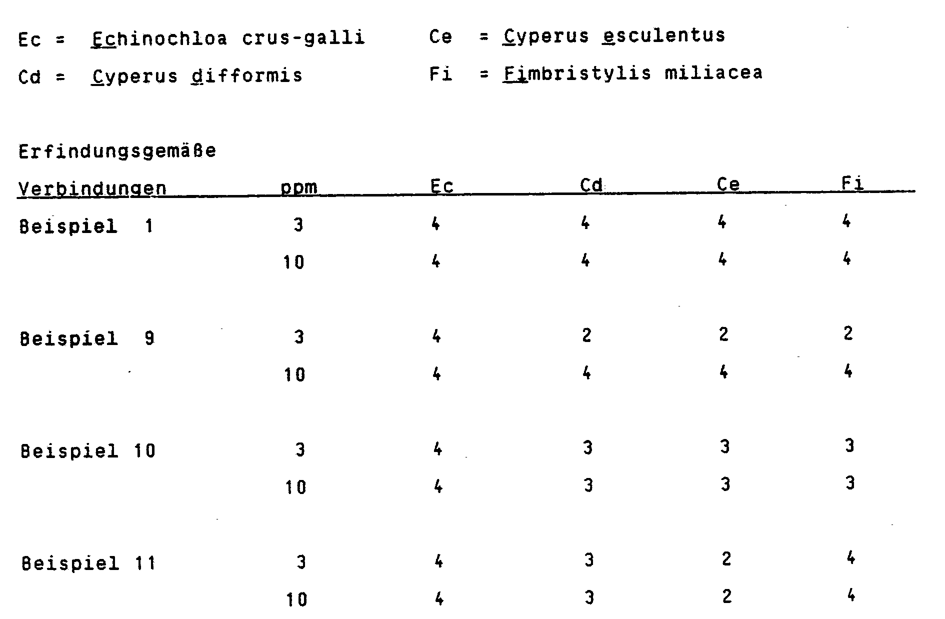

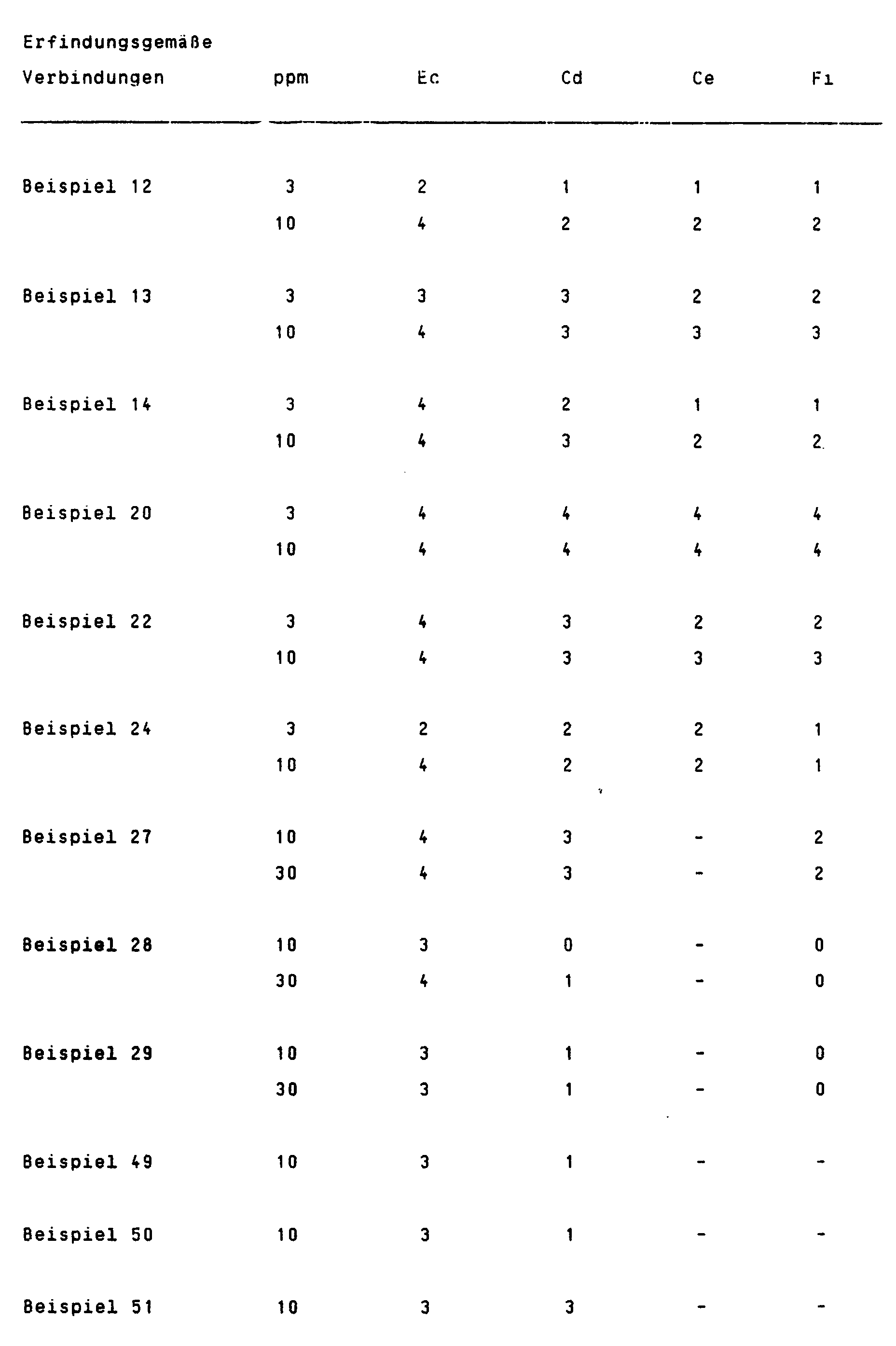

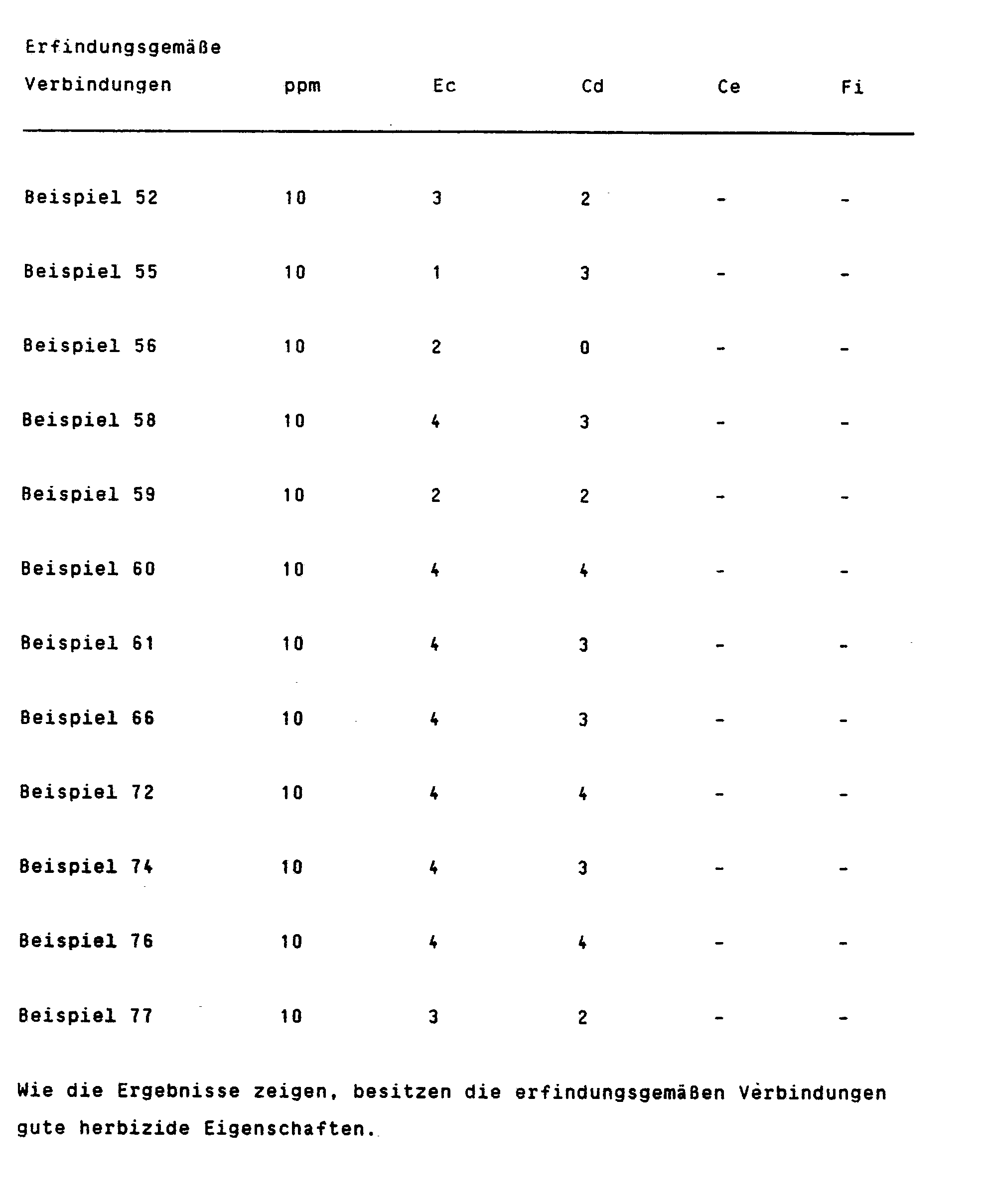

## Patentansprüche

1. Trifluormethylsubstituierte Azole der allgemeinen Formel I 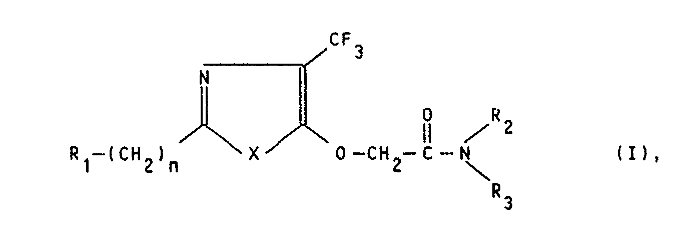 in der
R₁ einen gegebenenfalls ein- oder mehrfach, gleich oder verschieden durch Halogen, C₁₋₄-Alkoxy, C₁₋₄-Alkylthio oder Di-C₁₋₄-alkylamino substituierten C₁₋₁₂-Alkyl-, C₂₋₁₂-Alkenyl- oder C₂₋₁₂-Alkinylrest, einen C₃₋₈-Cycloalkylrest, einen C₁₋₆-Alkoxy-, C₁₋₆-Alkylthio- oder Di-C₁₋₆-alkylaminorest, einen gegebenenfalls ein- oder mehrfach, gleich oder verschieden durch Halogen, C₁₋₄-Alkyl, C₁₋₄-Alkoxy, C₁₋₄-Alkylthio, Di-C₁₋₄-alkylamino, Halogen-C₁₋₄-alkyl, Halogen-C₁₋₄-alkoxy, Halogen-C₁₋₄-alkylthio, C₁₋₆-Alkoxycarbonyl, Halogen-C₁₋₆-alkoxycarbonyl, Nitro, Cyan, Phenoxy, Halogenphenoxy, Phenylthio oder Halogenphenylthio substituierten Phenyl-, Biphenyl-, Naphthyl-, Furyl-, Thienyl-, Pyridyl-, Benzofuryl-, Benzothienyl-, Chinolinyl-, Isochinolinyl-, Phenoxy- oder Phenylthiorest,
R₂ und R₃ unabhängig voneinander ein Wasserstoffatom, einen gegebenenfalls ein- oder mehrfach, gleich oder verschieden durch Halogen, C₁₋₄-Alkoxy, C₁₋₄-Alkylthio oder Di-C₁₋₄-Alkylamino substituierten C₁₋₈-Alkyl-, C₂₋₈-Alkenyl- oder C₂₋₈-Alkinyl- rest, einen C₃₋₈-Cycloalkyl- oder C₃₋₈-Cycloalkenylrest, einen gegebenenfalls durch ein oder mehrere Sauerstoff-, Stickstoff- oder Schwefelatome unterbrochenen C₁₋₁₂-Alkyl-, C₃₋₈-Cycloalkyl-, C₃₋₈-Cycloalkenyl-, C₃₋₈-Cycloalkyl-C₁₋₄-alkyl- oder C₃₋₈-Cycloalkenyl-C₁₋₄-alkylrest, einen C₁₋₈-Alkoxyrest, einen gegebenenfalls ein- oder mehrfach, gleich oder verschieden durch Halogen, C₁₋₄-Alkyl, C₁₋₄-Alkoxy, C₁₋₄-Alkylthio, Di-C₁₋₄-alkylamino, Halogen-C₁₋₄-alkyl, Halogen-C₁₋₄-alkoxy, Halogen-C₁₋₄-alkylthio, C₁₋₆-Alkoxycarbonyl, Halogen-C₁₋₆-alkoxycarbonyl, Nitro, Cyan, Phenoxy, Halogenphenoxy, Phenylthio oder Halogenphenylthio substituierten Phenyl-, Biphenyl-oder Naphthylrest oder
R₂ und R₃ zusammen mit dem benachbarten Stickstoffatom einen gegebenenfalls weitere Heteroatome wie Sauerstoff, Stickstoff oder Schwefel enthaltenden und gegebenenfalls ein- oder mehrfach, gleich oder verschieden durch Halogen oder C₁₋₄-Alkyl substituierten gesättigten oder ungesättigten Monocyclus oder anellierten Bicyclus,
n 0, 1 oder 2,
X ein Sauerstoff- oder Schwefelatom oder die Gruppe NR₄ und
R₄ einen C₁₋₁₂-Alkylrest oder einen gegebenenfalls ein- oder mehrfach, gleich oder verschieden durch Halogen, C₁₋₄-Alkyl, C₁₋₄-Alkoxy, C₁₋₄-Alkylthio, Di-C₁₋₄-alkylamino, Halogen-C₁₋₄-alkyl, Halogen-C₁₋₄-alkoxy, Halogen-C₁₋₄-alkylthio, C₁₋₆-Alkoxycarbonyl, Halogen-C₁₋₆-alkoxycarbonyl, Nitro, Cyan, Phen oxy, Halogenphenoxy, Phenylthio, Halogenphenylthio substituierten Phenyl-, Biphenyl-, Naphthyl-, Furyl-, Thienyl-, Pyridyl-, Benzofuryl-, Benzothienyl-, Chinolinyl- oder Isochinolinylrest
bedeuten.

2. Trifluormethylsubstituierte Azole der allgemeinen Formel I gemäß Anspruch 1, dadurch gekennzeichnet, daß darin
R₁ einen gegebenenfalls ein- oder mehrfach, gleich oder verschieden durch Halogen, C₁₋₄-Alkoxy, C₁₋₄-Alkylthio oder Di-C₁₋₄-alkylaminosubstituierten C₁₋₁₂-Alkyl-, C₂₋₁₂-Alkenyl oder C₂₋₁₂-Alkinylrest, einen C₃₋₈-Cycloalkylrest, einen C₁₋₆-Alkoxy-, C₁₋₆-Alkylthio- oder Di-C₁₋₆-alkylaminorest, einen gegebenenfalls ein- oder mehrfach, gleich oder verschieden durch Halogen, C₁₋₄-Alkyl, C₁₋₄-Alkoxy, C₁₋₄-Alkylthio, Di-C₁₋₄-alkylamino, Halogen-C₁₋₄-alkyl, Halogen-C₁₋₄-alkoxy, Halogen-C₁₋₄-alkylthio, C₁₋₆-Alkoxycarbonyl, Halogen-C₁₋₆-alkoxycarbonyl, Nitro, Cyan, Phenoxy Halogenphenoxy, Phenylthio oder Halogenphylthio substituierten Phenyl-, Biphenyl-, Naphthyl-, Furyl-, Thienyl-, Pyridyl-, Benzofuryl-, Benzothienyl-, Chinolinyl-, Isochinolinyl-, Phenoxy- oder Phenylthiorest,
R₂ und R₃ unabhängig voneinander ein Wasserstoffatom, einen gegebenenfalls ein- oder mehrfach, gleich oder verschieden durch Halogen, C₁₋₄-Alkoxy, C₁₋₄-Alkylthio oder Di-C₁₋₄-Alkylamino substituierten C₁₋₈-Alkyl-, C₂₋₈-Alkenyl- oder C₂₋₈-Alkinyl- rest, einen C₃₋₈-Cycloalkyl- oder C₃₋₈-Cycloalkenylrest, einen gegebenenfalls durch ein oder mehrere Sauerstoff-, Stickstoff- oder Schwefelatome unterbrochenen C₁₋₁₂-Alkyl-, C₃₋₈-Cycloalkyl-, C₃₋₈-Cycloalkenyl-, C₃₋₈-Cycloalkyl-C₁₋₄-alkyl- oder C₃₋₈-Cycloalkenyl-C₁₋₄-alkylrest, einen C₁₋₈-Alkoxyrest, einen gegebenenfalls ein- oder mehrfach, gleich oder verschieden durch Halogen, C₁₋₄-Alkyl, C₁₋₄-Alkoxy, C₁₋₄-Alkylthio, Di-C₁₋₄-alkylamino, Halogen-C₁₋₄-alkyl, Halogen-C₁₋₄-alkoxy, Halogen-C₁₋₄-alkylthio, C₁₋₆-Alkoxycarbonyl, Halogen-C₁₋₆-alkoxycarbonyl, Nitro, Cyan, Phenoxy, Halogenphenoxy, Phenylthio oder Halogenphenylthio substituierten Phenyl-, Biphenyl-oder Naphthylrest oder
R₂ und R₃ zusammen mit dem benachbarten Stickstoffatom einen gegebenenfalls ein- oder mehrfach, gleich oder verschieden durch Halogen oder C₁₋₄-Alkyl subkstituierten Mono- oder Bicyclus der Formeln 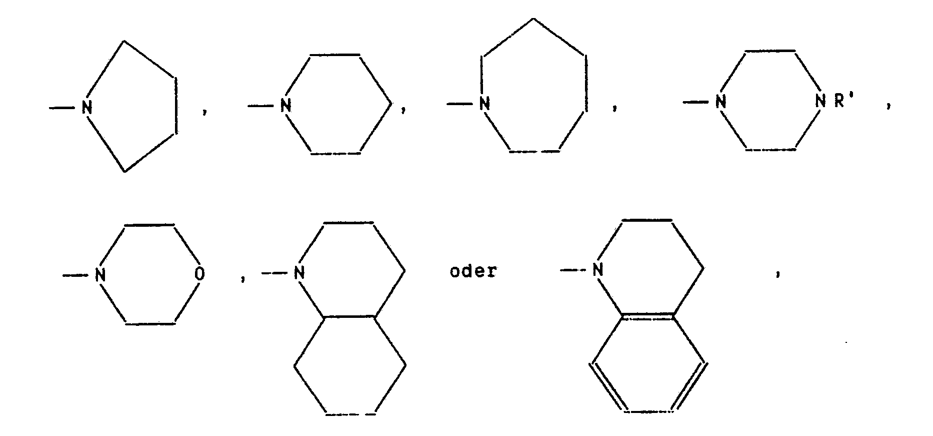 Rʹ ein Wasserstoffatom, einen C₁₋₄-Alkylrest, einen gegebenenfalls ein- oder mehrfach, gleich oder verschieden durch Halogen, C₁₋₄-Alkyl, C₁₋₄-Alkoxy, C₁₋₄-Alkylthio, Halogen-C₁₋₄-alkyl, Halogen-C₁₋₄-alkoxy, Halogen-C₁₋₄-alkylthio oder Nitro substituierten Phenyl- oder Benzylrest,
n 0, 1 oder 2,
X ein Sauerstoff- oder Schwefelatom oder die Gruppe NR₄ und
R₄ einen C₁₋₁₂-Alkylrest oder einen gegebenenfalls ein- oder mehrfach, gleich oder verschieden durch Halogen, C₁₋₄-Alkyl, C₁₋₄-Alkoxy, C₁₋₄-Alkylthio, Di-C₁₋₄-alkylamino, Halogen-C₁₋₄-alkyl, Halogen-C₁₋₄-alkoxy, Halogen-C₁₋₄-alkylthio, C₁₋₆-Alkoxycarbonyl, Halogen-C₁₋₆-alkoxycarbonyl, Nitro, Cyan, Phenoxy, Halogenphenoxy, Phenylthio, Halogenphenylthio substituierten Phenyl-, Biphenyl-, Naphthyl-, Furyl-, Thienyl-, Pyridyl-, Benzofuryl-, Benzothienyl-, Chinolinyl- oder Isochinolinylrest
bedeuten.

3. Trifluormethylsubstituierte Azole der allgemeinen Formel I gemäß Anspruch 1, dadurch gekennzeichnet, daß darin
R₁ einen Phenylrest, einen Halogenphenylrest, einen Methylphenylrest, einen Methoxyphenylrest oder einen Furylrest,
R₂ und R₃ unabhängig voneinander einen C₁₋₄-Alkylrest, einen C₁₋₄-Alkenylrest oder einen Phenylrest oder
R₂ und R₃ zusammen mit dem benachbarten Stickstoffatom einen gegebenenfalls durch C₁₋₄-Alkyl substituierten Piperidino- oder Hexahydroazepinylrest,
n 0,
X ein Sauerstoff- oder Schwefelatom oder die Gruppe NR₄ und
R₄ Dimethylphehyl
bedeuten.

4. Verfahren zur Herstellung von trifluormethylsubstituierten Azolen der allgemeinen Formel I, dadurch gekennzeichnet, daß man 5-fluor-4-(trifluormethyl)-substituierte Azole der allgemeinen Formel II 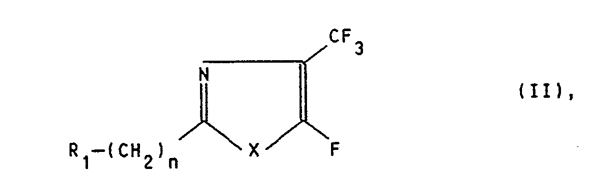 in der R_{₁}, X und n die oben genannten Bedeutungen haben, mit Hydroxyessigsäureamiden der allgemeinen Formel III 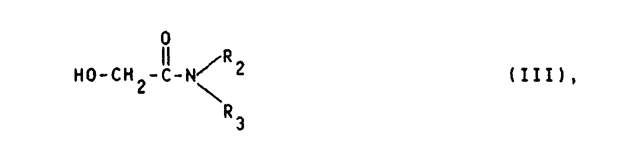 in der R₂ und R₃ die oben genannten Bedeutungen haben, umsetzt.

5. Mittel mit herbizider Wirkung, gekennzeichnet durch einen Gehalt an mindestens einer Verbindung der allgemeinen Formel I gemäß den Ansprüchen 1 bis 3.

6. Verwendung der Verbindungen der allgemeinen Formel I gemäß den Ansprüchen 1 bis 3 zur Bekämpfung von unerwünschtem Pflanzenwuchs in Nutzpflanzenkulturen.

7. Verfahren zur Herstellung von Mitteln mit herbizider Wirkung, dadurch gekennzeichnet, daß man Verbindungen der allgemeinen Formel I gemäß den Ansprüchen 1 bis 3 mit Träger- und/oder Hilfsstoffen vermischt.
